# EUROPÄISCHE PATENTANMELDUNG

(11) **EP 1 145 847 A1**
(43) Veröffentlichungstag der Anmeldung: **17.10.2001**
(21) Anmeldenummer: 01107490.3
(22) Anmeldetag: 29.03.2001
(51) Int. Cl.: B32B 27/40, B32B 7/02, B29C 47/06, A47C 27/08

(54) **Weichelastische Mehrschichtfolie und ihre Verwendung zur Herstellung geschlossener Füllkörper**

(30) Priorität: 10.04.2000 DE 10017817
(71) Anmelder: Wolff Walsrode AG, 29655 Walsrode (DE)
(72) Erfinder: Schledjewski, Ralf, Dr., 67663 Kaiserslautern (DE); Cassel, Antoine, 29664 Walsrode (DE); Brandt, Rainer, Dr., 29664 Walsrode (DE); Craig, Aileen, Dr., 29699 Bomlitz (DE)
(74) Vertreter: Pettrich, Klaus-Günter, Dr.

(57) **Zusammenfassung**

Beschrieben wird eine mehrschichtige Folie mit mindestens einer Schicht (1), aus thermoplastischen Polyurethan und mindestens einer weiteren Schicht (2), aus thermoplastischen Elastomer und gegebenenfalls einer weiteren Schicht (3) aus thermoplastischen Polyurethan, die mit Schicht (1) die Schicht (2) einschließt, dadurch gekennzeichnet, dass das Verhältnis der Wasserdampfdurchlässigkeiten der Schichten (1) und (2) mindestens zwei ist.

Ebenfalls beschrieben wird die Verwendung dieser Folie zur Herstellung von Füllkörpern wie insbesondere Wasserbetten oder Wärme-Kälte-Kissen.

## Beschreibung

Diese Erfindung betrifft mindestens zweischichtige elastische thermoplastische Folien, die aus mindestens einer Schicht aus thermoplastischem Polyurethan und mindestens einer weiteren Schicht bestehen, die aus einem thermoplastischen Elastomer mit geringer Wasserdampfdurchlässigkeit gebildet wird.

Die Kombination von Urethan-Polymer und einem thermoplastischen Elastomer ergibt eine weichelastische Folie mit hohem mechanischen Eigenschaftsprofil und einer sehr guten Wasserdampfsperrwirkung.

Für die Herstellung flexibler Füllkörper für gasförmige oder flüssige Medien, wie zum Beispiel Wasserbetten, werden weichelastische Folien mit guten mechanischen Eigenschaften benötigt.

Es ist bekannt, dass derartige Anwendungen mit Folien aus Weich-PVC bedient werden können. Aufgrund der spezifischen Eigenschaften des PVC ist dabei ein relativ hoher Materialeinsatz erforderlich.

Weiterhin ist bekannt, dass thermoplastische Polyurethane für die Herstellung flexibler Behälter für gasförmige oder flüssige Medien Verwendung finden. Vorteilhaft ist hier das hohe mechanische Eigenschaftsniveau, wodurch relativ geringe Wandstärken realisiert werden können. Problematisch stellt sich dabei der hohe Diffusionskoeffizient für polare Medien wie z.B. Wasserdampf dar.

Einschichtige Folien aus thermoplastischen Polyurethanen (TPE-U), Verfahren zu ihrer Herstellung sowie ihre Verwendung sind nach dem Stand der Technik beispielsweise aus der EP-A-0 308 683, der EP-A-0 526 858, der EP-A-0 571 868 oder der EP-A-0 603 680 bekannt. Die in diesen Schriften beschriebenen Aufbauten lassen sich als höherschmelzende Schicht bzw. Schichten in Dublofolien integrieren oder sind bereits in die nach ihrer Art bekannten Dublofolien integriert worden. Ebenso ist die Herstellung von TPE-U-Folien unter Einsatz von im wesentlichen unverträglichen Polymeren als Mattierungsmittel in TPE-U z.B. in der DE-A 41 26 499 beschrieben.

Stand der Technik sind auch mehrschichtige, durch Coextrusion hergestellte Folien aus TPE-U und anderen, der Gruppe der Thermoplaste zugeordneten, Rohstoffen. Neben der Coextrusion mit polyolefinischen Thermoplasten, bei denen in der Regel die polyolefinische Schicht praktisch keine Verbundhaftung zur TPE-U-Schicht eingeht und lediglich die Funktion einer Stütz- beziehungsweise Trennschicht besitzt, sind auch mehrschichtige Aufbauten mit guter Verbundhaftung bekannt. In EP-A-0 842 768 wird beispielsweise ein mehrschichtiger Aufbau aus TPE-U und einem polyolefinischen Haftvermittler beschrieben. Bei derartigen Aufbauten kommt es aufgrund der geringen Elastizität der Thermoplaste bei häufigeren Lastwechseln mit hohen Dehnungswerten zu Rissbildungen in der Thermoplast-Schicht und somit zu einem Funktionsverlust.

Neben thermoplastischen Polyurethanen existieren weitere weichelastische Materialien die allgemein unter dem Oberbegriff thermoplastische Elastomere (TPE) zusammengefasst sind. Zu den für die Folienverarbeitung geeigneten Gruppen gehören primär die Blockcopolymere. Dies sind neben den TPE-U die Styrol-basierenden Systeme (TPE-S), die Polyetherestertypen (TPE-E) und die Polyetherblockamide (TPE-A). Folien auf Basis von TPE-E werden beispielsweise in US 5 859 083 beschrieben, wobei hier insbesondere auf die hohe Wasserdampfdurchlässigkeit abgehoben wird. Gleiches gilt für die in EP-A-0761 715 beschriebenen Folien aus TPE-A. Foliengeeignete TPE-S und deren Verwendung werden beispielsweise in DE-A-1 9628 834 beschrieben. Eine Übersicht über die Gruppe der thermoplastischen Elastomere geben beispielsweise:

Thermoplastic elastomers: a comprehensive review, ed. N.R. Legge, G. Holden and H. E. Schroeder, Carl Hanser Verlag, München, 1987 und Thermoplastische Elastomere - Herausforderung an die Elastomerverarbeiter, Hrsg.: VDI-Gesellschaft Kunststofftechnik, VDI Verlag, Düsseldorf, 1997.

Es stellte sich somit die Aufgabe eine weichelastische Folie mit einem mechanischen Eigenschaftsprofil, das dem von Weich-PVC überlegen ist, kombiniert mit einer hohen Wasserdampfundurchlässigkeit bereitzustellen. Zur Minimierung der bei der Fertigung anfallenden Kosten sollte die Kombination der Eigenschaften nach Möglichkeit in einem Einstufen-Prozess erfolgen.

Erfindungsgemäß gelang es, eine den genannten Anforderungen genügende Folie bereitzustellen, die dadurch gekennzeichnet ist, dass die Folie aus mindestens zwei Schichten aufgebaut ist, wobei mindestens eine Schicht aus thermoplastischen Polyurethan (1) besteht. Die Folie wird weiterhin aufgebaut aus mindestens einer weiteren Schicht (2), die aus thermoplastischen Elastomer gebildet wird, die dadurch gekennzeichnet ist, dass das Verhältnis der Wasserdampfdurchlässigkeiten der Schichten (1) und (2) mindestens zwei ist.

Überraschenderweise gehorcht die Wasserdampfdurchlässigkeit des Verbundes bei dem genannten Verhältnis von mindestens zwei nicht linearen Gesetzmäßigkeiten. Wird das Verhältnis der Wasserdampfdurchlässigkeiten der Schichten (1) und (2) auf mindestens sechs erhöht, so kann die Wasserdampfdurchlässigkeit der Mehrschichtfolien minimiert werden. Deshalb beträgt in einer bevorzugten Ausführung der Mehrschichtfolie das Verhältnis der Wasserdampfdurchlässigkeiten der Schichten (1) und (2) mindestens sechs.

Die Schicht (1) der erfindungsgemäßen Mehrschichtfolie ist aus wenigstens einem thermoplastischen Polyurethanelastomeren aufgebaut, vorzugsweise aus einem überwiegend linearen thermoplastischen Polyurethanelastomeren, dessen längerkettige Diolkomponente ein Polyester oder Polyether ist, und die eine Shore-Härte von vorzugsweise 70 - 95 A, besonders bevorzugt 85 - 95 A, bestimmt nach DIN 53 505, aufweist.

Geeignete thermoplastische Polyether- oder Polyester-Polyurethan-Elastomere und/oder Mischungen derselben können beispielsweise durch die bekannten Batch- und/oder teil- und/oder vollkontinuierlichen Verfahren, insbesondere aber durch Umsetzung in einem Schneckenwerkzeug von
a) organischen, vorzugsweise aromatischen oder cycloaliphatischen Diisocyanaten,
b) polymeren Diolen mit Molekulargewichten von bevorzugt 500 bis 8000,
c) Kettenverlängerungskomponenten mit Molekulargewichten von vorzugsweise 60 bis 400 in Gegenwart von gegebenenfalls
d) Katalysatoren sowie
e) Hilfsstoffen und/oder Zusätzen
hergestellt werden.

Über die verwendbaren Komponenten a) bis e) lässt sich folgendes ausführen:
a) Als organische Diisocyanate (a) kommen vorzugsweise aromatische oder cycloaliphatische Diisocyanate in Betracht. Im einzelnen seien beispielhaft genannt: aromatische Diisocyanate, wie 2,4- und 2,6-Toluylen-Diisocyanat, 4,4'-, 2,4'- und 2,2'-Diphenylmethan-Diisocyanat oder Gemische daraus, cycloaliphatische Diisocyanate, wie Isophoron-Diisocyanat, 1,4-Cyclo-hexan-Diisocyanat sowie 4,4'-, 2,4'- und 2,2'-Dicyclo-hexylmethan-Diisocyanat oder Gemische daraus.
b) Als höhermolekulare Diolverbindungen (b) mit bevorzugten Molekulargewichten von 400 bis 8000 eignen sich vorzugsweise linear aufgebaute Moleküle mit einer niedrigen Glas- oder Erweichungstemperatur. Hierzu zählen die Polyetherole und Polyesterole. In Betracht kommen aber auch hydroxylgruppen-haltige Polymere, beispielsweise Polyacetale, wie Polyoxymethylene und vor allem wasserunlösliche Formale und aliphatische Polycarbonate, insbesondere solche aus Diphenylcarbonat und Hexandiol-1,6, hergestellt durch Umesterung. Außerdem kommen hydroxylgruppenverkappte Diolverbindungen aus Polyolefinen, insbesondere aliphatische hydroxylgruppenverkappte Copolymere aus Ethylen und Butylen, in Betracht. Die Diolverbindungen müssen zumindest überwiegend linear, d.h. im Sinne der Isocyanatreaktion difunktionell aufgebaut sein. Die genannten Diolverbindungen können als Einzelkomponenten oder in Form von Mischungen zur Anwendung kommen.
c) Als Kettenverlängerungsmittel mit Molekulargewichten von 60 bis 400, vorzugsweise 60 bis 300, kommen bevorzugt Alkandiole mit 2 bis 12 Kohlenstoffatomen, vorzugsweise mit 2,4 oder 6 Kohlenstoffatomen, wie z.B. Ethandiol, Hexandiol-1,6, und insbesondere Butandiol-1,4 und Dialkylenetherglycole wie z.B. Diethylenglycol und Dipropylenglycol in Betracht. Geeignet sind jedoch auch Diester der Terephthalsäure mit Alkandiolen mit 2 bis 4 Kohlenstoffatomen, wie z.B. Terephthalsäure-bis-ethandiol oder - butandiol-1,4, Hydroxyalkylenether des Hydrochinons, (cyclo-) aliphatische Diamine, wie z.B. Isophorondiamin, Ethylendiamin und aromatische Diamine, wie z.B. 2,4- und 2,6-Toluylendiamin.
d) Geeignete Katalysatoren, welche insbesondere die Reaktion zwischen den Isocyanatgruppen der Kategorie a) und den Hydroxylgruppen der Kategorien b) und c) beschleunigen, sind die nach dem Stand der Technik bekannten und üblichen tertiären Amine, wie z.B. Triethylamin, Dimethylcyclohexylamin, N-Methylmorpholin, N,N'-dimethylpeperazin, 2-(Dimethylamino-ethoxy)-ethanol, Diazabicyclo(2,2,2)-octan und ähnliche sowie insbesondere organische Metallverbindungen wie Titansäureester, Eisenverbindungen wie z.B. Eisen-(III)-acetylacetonat, Zinn-verbindungen, z.B. Zinndiacetat, Zinndioctoat, Zinndilaurat oder die Zinndialkylsalze aliphatischer Carbonsäuren wir Dibutyl-zinndiacetat, Dibutylzinndilaurat oder ähnliche. Die Katalysatoren werden üblicherweise in Mengen von 0,001 bis 0,2 Teilen pro 100 Teile Hydroxylverbindung b) eingesetzt.
e) Neben Katalysatoren können den Aufbaukomponenten a) bis c) auch Hilfsmittel und/oder Zusatzstoffe e) beigegeben sein. Genannt seien beispielsweise Gleitmittel, Inhibitoren, Stabilisatoren gegen Hydrolyse, Licht, Hitze, Oxydation oder Verfärbung, Farbstoffe, Pigmente, anorganische und/oder organische Füllstoffe, Verstärkungsmittel und niedermolekulare Weichmacher.

Geeignete thermoplastische Polyurethane sind beispielsweise unter den Handelsnamen Desmopan, Elastollan, Estane, Morthane, Pellethane, Pearlthane, Skythane, Tecoflex oder Texin erhältlich.

In einer besonders geeigneten Ausführung weisen die erfindungsgemäßen Folien elastische Urethan-Elastomer-Rezepturkomponenten auf, deren Weichsegment-Phase zu einem überwiegenden Teil aus Ether-Weichsegment-Bausteinen gebildet wird. Hierdurch wird eine bessere Beständigkeit gegen Hydrolyse erreicht. Zudem weisen derartige Materialien eine bessere Resistenz gegen Pilz- und Mikrobenbefall auf.

Zur Herstellung der Schicht (2) werden vorzugsweise thermoplastische Elastomere mit einer Wasserdampfdurchlässigkeit eingesetzt, die zwischen 0 und 150g/cm²*d liegt, gemessen an einer 50µm Schicht nach DIN 53122 bei 23°C und 85% relativer Feuchte.

In einer besonders bevorzugten Ausführung der erfindungsgemäßen Folie weist das zur Herstellung der Schicht (2) verwendete thermoplastische Elastomer eine Wasserdampfdurchlässigkeit zwischen 0 und 50g/cm²*d auf, gemessen an einer 50µm Schicht nach DIN 53122 bei 23°C und 85% relativer Feuchte.

In einer noch bevorzugteren Ausführung der erfindungsgemäßen Folie weist das zur Herstellung der Schicht (2) verwendete thermoplastische Elastomer eine Wasserdampfdurchlässigkeit zwischen 0 und 20g/cm²*d auf, gemessen an einer 50µm Schicht nach DIN 53122 bei 23°C und 85% relativer Feuchte.

In einer besonders bevorzugten Ausführung werden in der Schicht (2) der erfindungsgemäßen Folie styrolbasierende thermoplastische Elastomere (TPE-S) eingesetzt.

Die erfindungsgemäß eingesetzten TPE-S bestehen aus abwechselnden Blöcken oder Segmenten, die aus Styrol-Monomer-basierenden Einheiten, auch als Hartsegment referenziert, und weicheren, gummiartigen Einheiten, den sogenannten Weichsegmenten aufgebaut sind. Die einzelnen Blöcke bestehen dabei üblicherweise aus mindestens hundert Monomereinheiten. Weit verbreitet sind linear aufgebaute Triblock-Strukturen Styrol- / Weichsegment- / Styrol-Block. Ebenso gibt es lineare, sternförmige und verzweigte Strukturen basierend auf einzelnen oder wiederholt eingebauten Blöcken des Typs (Styrol- / Weichsegment -Block)n, mit n größer oder gleich 1.

Im Festkörper bilden diese Polymere mehrere Phasen aus. So liegen die Polystyrol-Blöcke vor und nach der Verarbeitung assoziiert in steifen Domänen vor. "Physikalische Vernetzung" durch diese Domänen führt zu einen kontinuierlichen dreidimensionalen Netzwerk. Während der Verarbeitung, allgemein unter der Einwirkung von Wärme oder Scherung, erweicht die Polystyrol-Hartphase und ermöglicht das Fließen des Materials unter Auflösung der vormaligen Domänen. Nach der Verarbeitung bilden sich während des Abkühlvorgangs erneut die Hartsegmentdomänen aus, die somit die weiche Phase an Fließvorgängen hindern. Diese thermisch initiierbaren Eigenschaftsveränderungen können mehrfach durchlaufen werden und erlauben somit vielfache Prozessschritte mit diesen Materialien.

Die gummiartige Weichphase steuert Elastizität bzw. Weichheit zu den Materialeigenschaften bei, während die "physikalische Vernetzung" und die Wechselwirkungen zwischen den Styrol-Blöcken die Festigkeit des Styrol-Elastomeren gewährleisten.

Eine geeignete Ausführung der erfindungsgemäßen Folien enthält in den Schichten (1) und (2) zusätzlich gebräuchliche Additive aus der Gruppe umfassend:
I. Antiblockmittel, anorganische oder organische Abstandshalter,
II. Gleit- oder Entformungsmittel,
III. Pigmente oder Füllstoffe und
IV. Stabilisatoren.

Der Anteil der genannten Additive I bis IV liegt in Summe bevorzugt zwischen 0% und 30%.

Die gebräuchlichen Additive, die in den erfindungsgemäßen Folien enthalten sein können, sind beispielsweise bei Gächter und Müller beschrieben in: Kunststoff-Additive, Carl Hanser Verlag München, 3. Ausgabe (1989).

Ein mindestens dreischichtiger Aufbau der Mehrschichtfolie, dadurch gekennzeichnet, dass die das thermoplastische Elastomer enthaltende Schicht (2) mindestens zwischen zwei aus thermoplastischem Polyurethan aufgebauten Schichten (1) liegt stellt eine besonders bevorzugte Ausführung der erfindungsgemäßen Folie dar.

Erfindungsgemäß bevorzugt sind Folien mit einer Gesamtdicke zwischen 50 µm und 600 µm.

Bei einer solchen Folie liegen die Dicke der Schicht (1) aus thermoplastischen Polyurethanen bevorzugt zwischen 20 µm und 400 µm, die Dicke der TPE-Schicht (2) bevorzugt zwischen 10 µm und 200 µm.

Zur Herstellung der erfindungsgemäßen Mehrschichtfolie eignen sich besonders die gängigen thermischen Umformverfahren zur Verarbeitung von Kunststoffen zu mehrschichtigen Flächengebilden. Hier wäre die Herstellung durch Coextrusion zu nennen, die bevorzugt nach dem Blasfolienverfahren erfolgt. Aufgrund der besseren erzielbaren Verbundhaftung ist die Coextrusion unter den geeigneten Herstellungsverfahren von mehrschichtigen thermoplastischen Flächengebilden im besonderen Maß bevorzugt.

Die erfindungsgemäßen Folien können mit den bekannten physikalischen und chemischen Behandlungsmethoden wie beispielsweise der Corona-Behandlung ein- oder beidseitig in ihren Oberflächeneigenschaften modifiziert werden.

Ein weiterer Gegenstand der Erfindung ist deren Verwendung zur Herstellung von Füllkörpern, wobei das Füllgut sowohl flüssiger als auch gasförmiger Natur sein kann. Insbesondere eignen sich derartige Füllkörper zur Aufnahme von Wasser. Die Herstellung der Füllkörper erfolgt unter Einsatz gängiger Fügeverfahren, wie z.B. Hochfrequenzschweißen.

Erfindungsgemäß besonders bevorzugt ist der Einsatz der Folie zur Herstellung von Füllkörpern wie Wasserbetten oder Wärme-Kälte-Kissen.

### Beispiele

Die im Rahmen der nachfolgenden Beispiele und Vergleichsbeispiele beschriebenen Folien wurden durch Blasfoliencoextrusion hergestellt. Die zum Aufschluss thermoplastischer Harze geeigneten Schneckenwerkzeuge sind in ihrem Aufbau z.B. von Wortberg, Mahlke und Effen in: Kunststoffe, 84 (1994) 1131-1138, von Pearson in: Mechanics of Polymer Processing, Elsevier Publishers, New York, 1985 oder der Fa. Davis-Standard in: Paper, Film & Foil Converter 64 (1990) S. 84- 90 beschrieben. Werkzeuge zum Ausformen der Schmelze zu Folien sind u.a. von Michaeli in: Extrusions-Werkzeuge, Hanser Verlag, München 1991 erläutert.

### Beispiel 1

Mit Hilfe eines Zweischicht-Blasfolienwerkzeuges wurde eine Folie hergestellt, deren Schicht (1) aus einem Ether-TPE-U der Shore-A-Härte 87, gemessen nach DIN 53 505, entsprechend einer Härte von 36 Shore-D, gebildet wurde. Dieser 100 µm dicken Schicht wurden als Additive 2% Abstandshalter und 0,5% Wachse zugesetzt. Sämtliche für diese Schicht eingesetzten Komponenten wurden gemeinsam in einem Extruder aufgeschmolzen.

Die 100µm Schicht (2) wurde aus einem TPE-S hergestellt. Als TPE-S wurde ein Styrol-Butadien-Styrol Blockcopolymer der Shore-A-Härte 85, gemessen nach DIN 53 505, entsprechend einer Härte von 30 Shore-D, verwendet.

Die Extrusionseinrichtungen wurden mit Temperaturen zwischen 170°C und 200°C betrieben. Die beiden Schmelzeströme wurden in einem Zweischicht-Blasfolienkopf mit einer Verarbeitungstemperatur von 200°C übereinandergelegt und durch eine Ringspaltdüse mit einem Durchmesser von 150 mm ausgetragen. Durch Anblasen mit Luft wurde die ringförmige Schmelzefahne abgekühlt, anschließend flachgelegt, getrennt und aufgewickelt.

### Beispiel 2

Mit Hilfe eines Dreischicht-Blasfolienwerkzeuges wurde eine Folie hergestellt, deren beiden Außenschichten (1) und (3) aus einem Ether-TPE-U der Shore-A-Härte 87, gemessen nach DIN 53 505, entsprechend einer Härte von 36 Shore-D, gebildet wurde. Diesen 75 µm dicken Schichten wurden als Additive 4% Abstandshalter und 1% Wachse zugesetzt. Sämtliche für jede dieser Schichten eingesetzten Komponenten wurden gemeinsam in einem Extruder aufgeschmolzen.

Die 50µm Schicht (2) wurde aus einer haftvermittelnden Komponente hergestellt. Als haftvermittelnde Komponente wurde ein Copolymerisat aus Ethylen und Maleinsäureanhydrid mit einem Maleinsäureanhydrid-Anteil unter 5 Gew.-% eingesetzt. Die Härte dieser zum Aufbau der haftvermittelnden Schicht verwendeten Komponente betrug ca. 67 Shore-A, gemessen nach DIN 53 505, entsprechend ca. 15 Shore-D.

Die Extrusionseinrichtungen wurden mit Temperaturen zwischen 160°C und 200°C betrieben. Die drei Schmelzeströme wurden in einem Blasfolienkopf mit einer Verarbeitungstemperatur von 195°C übereinandergelegt und durch eine Ringspaltdüse mit einem Durchmesser von 130 mm ausgetragen. Durch Anblasen mit Luft wurde die ringförmige Schmelzefahne abgekühlt, anschließend flachgelegt, getrennt und aufgewickelt.

### Beispiel 3

Mit Hilfe eines Einschicht-Blasfolienwerkzeuges wurde eine Folie hergestellt, deren Schicht (1) aus einem Ether-TPE-U der Shore-A-Härte 87, gemessen nach DIN 53 505, entsprechend einer Härte von 36 Shore-D, gebildet wurde. Dieser 200 µm dicken Schicht wurden als Additive 2% Abstandshalter und 0,3% Wachse zugesetzt. Sämtliche für diese Schicht eingesetzten Komponenten wurden gemeinsam in einem Extruder aufgeschmolzen.

Die Extrusionseinrichtung wurde mit Temperaturen zwischen 170°C und 200°C betrieben. Der Schmelzestrom wurden mit einer Verarbeitungstemperatur von 200°C durch eine Ringspaltdüse mit einem Durchmesser von 150 mm ausgetragen. Durch Anblasen mit Luft wurde die ringförmige Schmelzefahne abgekühlt, anschließend flachgelegt, getrennt und aufgewickelt.

### Beispiel 4

Mit Hilfe eines Einschicht-Blasfolienwerkzeuges wurde eine Folie hergestellt, deren Schicht (1) aus einem Styrol-Butadien-Styrol Blockcopolymer der Shore-A-Härte 85, gemessen nach DIN 53 505, entsprechend einer Härte von 30 Shore-D, gebildet wurde.

Die Extrusionseinrichtung wurde mit Temperaturen zwischen 170°C und 200°C betrieben. Der Schmelzestrom wurde mit einer Verarbeitungstemperatur von 195°C durch eine Ringspaltdüse mit einem Durchmesser von 150 mm in einer Schichtdicke von 100µm ausgetragen. Durch Anblasen mit Luft wurde die ringförmige Schmelzefahne abgekühlt, anschließend flachgelegt, getrennt und aufgewickelt.

### Bewertung der im Rahmen der Beispiele hergestellten Folien:

Alle vier Folien wurden hinsichtlich Wasserdampfdurchlässigkeit, gemessen nach DIN 53 122 bei 23°C und 85% relativer Feuchte, und mechanischer Belastbarkeit, gemessen nach DIN EN ISO 527 (Zugversuch) und DIN 53 515 (Weiterreißfestigkeit) charakterisiert. An den beiden Mehrschichtfolien (Beispiel 1 und Beispiel 2) wurde die Verbundhaftung, gemessen nach DIN 53 357 Verfahren B, der Folien im frischen Zustand und nach Ermüdungsbelastung bestimmt. Die Ermüdungsbelastung erfolgte in Form einer dynamischen Wechselbelastung mit einer Dehnungsamplitude von 50% über 10 000 Zyklen.

| | | **Beispiel 1** | **Beispiel 2** | **Beispiel 3** | **Beispiel 4** |
|---|---|---|---|---|---|
| Dicke | [µm] | 195 | 195 | 205 | 100 |
| Wasserdampfdurchlässigkeit DIN 53 122 bei 23°C/85% rel.F. | [g/m²•d] | 11 | 8,2 | 132,5 | 20 |
| Bruchspannung DIN EN ISO 527 | [MPa] | 44,9 | 48,5 | 54,3 | 28 |
| Bruchdehnung DIN EN ISO 527 | [%] | 723 | 858 | 823 | 605 |
| Weiterreißfestigkeit DIN 53 515 | [N/mm] | 51,7 | 42 | 55,2 | 32 |
| Verbundhaftung (frisch) DIN 53 357 Verf. B | [N/15mm] | >10 | 6,13 | -- | -- |
| Verbundhaftung nach Ermüdungsbelastung DIN 53 357 Verf. B | [N/15mm] | >10 | 0 | -- | -- |

Es ist klar zu erkennen, dass durch den erfindungsgemäßen Folienaufbau aus Beispiel 1 eine deutliche Reduktion der Wasserdampfdurchlässigkeit erzielt wird, die sich nicht linear aus den Wasserdampfdurchlässigkeiten der einzelnen Schichten (Beispiel 3 und Beispiel 4) herleiten lässt. Gleichzeitig bleiben die mechanischen Eigenschaften auf hohem Niveau erhalten. Beispiel 2 zeigt, dass bei der Verwendung von nicht erfindungsgemäßen Materialien, hier ein thermoplastischer Haftvermittler, in der Schicht (2) deutliche Defizite in den Langzeiteigenschaften gegeben sind.

## Patentansprüche

1. Mehrschichtige Folie mit mindestens einer Schicht (1), aus thermoplastischen Polyurethan und mindestens einer weiteren Schicht (2), aus thermoplastischen Elastomer und gegebenenfalls einer weiteren Schicht (3) aus thermoplastischen Polyurethan, die mit Schicht (1) die Schicht (2) einschließt, **dadurch gekennzeichnet, dass** das Verhältnis der Wasserdampfdurchlässigkeiten der Schichten (1) und (2) mindestens zwei ist.

2. Folie gemäß Anspruch 1, **dadurch gekennzeichnet, dass** das Verhältnis der Wasserdampfdurchlässigkeiten der Schichten (1) und (2) mindestens sechs ist.

3. Folie gemäß einem der vorhergehenden Ansprüche, **dadurch gekennzeichnet, dass** die Wasserdampfdurchlässigkeit der Schicht (2) zwischen 0 und 150g/cm²*d liegt, gemessen an einer 50µm Schicht nach DIN 53122 bei 23°C und 85% relativer Feuchte.

4. Folie gemäß einem der vorhergehenden Ansprüche, **dadurch gekennzeichnet, dass** die Wasserdampfdurchlässigkeit der Schicht (2) zwischen 0 und 50g/cm²*d auf, gemessen an einer 50µm Schicht nach DIN 53122 bei 23°C und 85% relativer Feuchte.

5. Folie gemäß einem der vorhergehenden Ansprüche, **dadurch gekennzeichnet, dass** die Wasserdampfdurchlässigkeit der Schicht (2) zwischen 0 und 20g/cm²*d auf, gemessen an einer 50µm Schicht nach DIN 53122 bei 23°C und 85% relativer Feuchte.

6. Folie nach einem der vorhergehenden Ansprüche, **dadurch gekennzeichnet, dass** das in der Schicht (1) eingesetzte thermoplastische Polyurethan ein im wesentlichen durch Ethergruppen aufgebautes Weichsegment besitzt.

7. Folie nach einem der vorhergehenden Ansprüche, **dadurch gekennzeichnet, dass** die Schicht (3) formuliert ist wie die Schicht (1).

8. Folie nach einem der vorangehenden Ansprüche, **dadurch gekennzeichnet, dass** das thermoplastischen Elastomer der Schicht (2) ein styrolbasierendes thermoplastisches Elastomer ist.

9. Folie nach einem der vorangehenden Ansprüche, **dadurch gekennzeichnet, dass** sie eine Gesamtdicke zwischen 50 µm und 600 µm hat, wobei die Dicke der Schicht(en) aus thermoplastischen Polyurethanen zwischen 20 µm und 400 µm, und die Dicke der thermoplastischen Elastomer-Schichten zwischen 10 µm und 200 µm beträgt.

10. Verwendung einer mehrschichtigen Folie gemäß einem der vorherigen Ansprüche zur Herstellung von geschlossenen Füllkörpern.
